# EUROPEAN PATENT APPLICATION

(11) **EP 2 767 310 A1**
(43) Date of publication of application: **20.08.2014**
(21) Application number: 14000138.9
(22) Date of filing: 13.04.2010
(51) Int. Cl.: A61P 3/00, A61P 25/00, A61K 31/137, A61P 3/04, A61P 25/22, A61P 25/24, A61P 43/00, A61P 25/30, A61P 25/28

(54) **Methods Of Reduction Of Interpatient Variability**

(30) Priority: 13.04.2009 US 422834
(62) Divisional of application: 10765032.7
(71) Applicant: Auspex Pharmaceuticals, Inc., Vista, CA 92081 (US)
(72) Inventor: Gant, Thomas G., La Jolla, CA 92037 (US); Sarshar, Sepehr, La Jolla, CA 92037 (US)
(74) Representative: Müller-Boré & Partner Patentanwälte PartG mbB

(57) **Abstract**

Disclosed herein are methods for the reduction of inter-patient variability of a drug, such as in one or more parameters including half-lite (t _{½}), maximum plasma concentration (Cₘₐₓ), time at maximal plasma concentration (Tₘₐₓ), area under the time-versus-concentration curve (AUC). Also provided are methods of reducing side effects associated with drugs.

## Description

This application claims the benefit of priority of United States Application No. 12/422,834, filed April 13, 2009, which is a continuation-in-part of United States Application No. 12/234,236, filed September 19, 2008, which is a continuation of 11/565,451, filed November 30, 2006, which claims the benefit of U.S. Provisional Application No. 60/741,315, filed December 1, 2005, and U.S. Provisional Application No. 60/841,366, filed August 30, 2006; this application is also a continuation-in-part of United States Application No. 11/598,572, filed November 13, 2006, which claims the benefit of U.S. Provisional Application No. 60/736,581, filed November 14, 2005, and U.S. Provisional Application No. 60/741,530, filed December 1, 2005; this application is also a continuation-in-part of United States Application No. 11/544,407, filed October 4, 2006, which claims the benefit of U.S. Provisional Application No. 60/724,160, filed October 6, 2005, and U.S. Provisional Application No. 60/741,316, filed December 1, 2005; this application is also a continuation-in-part of United States Application No. 11/562,890, filed October 4, 2006, which claims the benefit of U.S. Provisional Application No. 60/739,261, filed November 23, 2005, and U.S. Provisional Application No. 60/837,830, filed August 11, 2006; all of which are incorporated by reference as if written herein in their entireties.

Interpatient variability poses a daunting problem to the medical practitioner, pharmaceuticals developer, and individual patient alike. For example, differences in the way each patient absorbs, metabolizes, and clears drugs from the system result in differences in efficaciousness of the drug, length of activity, side effects, and interactions with other medications. For this reason, even a doctor's sound medical advice may not yield the optimal choice of drug or dosage upon the first prescription, or even upon subsequent ones. As archaic as it sounds, a certain amount of trial and error is required of each patient. Adverse side effects may cause a patient to discontinue a medication if the perceived quality of life seems reduced, even if debilitating and burdensome disease persists. Likewise, medications which are rapidly cleared from the system of a sub-population of patients, requiring multiple doses per day, are known to have lower patient compliance, and a correspondingly lower preference among physicians for prescription. This occurs even when the other properties of the drug would make it a better choice for the patient. Medications which possess the property of reduced interpatient variability would therefore find greater acceptance with doctors and patients and result in an overall improvement in public health. This improvement would manifest in both a nearer-to-optimal treatment efficacy in a proportion of patients, and better overall predictability in the larger population.

The bioavailability of a drug to a patient is determined to a certain extent by the dissolution rate and absorption of the drug in the GI tract if the drug is orally formulated, or by the permeability and absorption of the drug if delivered topically or transdermally/ transmucosally. In a common scenario, a drug candidate molecule is too poorly absorbed or too quickly excreted to be of much use as a pharmaceutical. It is quite common in modern medicinal chemistry to engineer analogues of active molecules in order to improve these phannacokinetic properties. Additionally, specialized formulations may be developed to achieve the same purposes - examples include the use of enteric coatings on tablets in order to target the distribution of drug to lower portions of the GI tract, and the use of polymer matrices as excipients to effect sustained release. Once in the bloodstream or target tissue, the drug molecule may experience a variety of fates including distribution to its locus of action (the desired fate) as well as tissue reservoirs, binding to serum proteins, metabolism (or biotransformation) and, ultimately, clearance.

It is metabolism of the drug, though, that presents the crux of the challenge to the pharmaceuticals developer. The body possesses remarkably efficient mechanisms for the clearance of drugs once they have entered circulation. The biotransformation, or metabolism, of a drug occurs by reaction with enzymes which alter the chemical structure of drugs and toxins in order to prepare them for clearance, and may proceed in two phases. Phase I reactions (also termed non-synthetic reactions) may occur by oxidation, reduction, hydrolysis, cyclization, and decyclization reactions. Oxidation involves the enzymatic addition of oxygen or removal of hydrogen, carried out by mixed function oxidases, often in the liver. These oxidative reactions typically involve a cytochrome P₄₅₀ hemoprotein, NADPH and oxygen. Alterations to the parent molecule are small and typically solubilizing, and frequently result in active metabolites. Phase II reactions - usually known as conjugation reactions (e.g., glucuronidation, sulfation, or conjugation of glutathione or amino acids) - are usually detoxication in nature, and involve the formation of a covalent bond between the polar functional groups of phase I metabolites and the conjugate (e.g., glucuronic acid, sulfate, etc.). Products of conjugation reactions have increased molecular weight and are usually inactive. Sites on drugs where conjugation reactions occur include carboxyl (-COOH), hydroxyl (-OH), amino (NH₂), and sulfhydryl (-SH) groups.

Among the phase I biotransforming enzymes, the cytochrome P₄₅₀ system ranks first in terms of catalytic versatility and the sheer number of xenobiotics (drugs or other exogenous substances) it detoxifies or activates to reactive intermediates. Human cytochrome P₄₅₀ enzymes are primarily membrane-associated proteins, located either in the inner membrane of mitochondria or in the endoplasmic reticulum of cells, but CYPs can be found in virtually all tissues. Liver microsomal CYPs play a very important role in determining the intensity and duration of action of drugs, and they also play a key role in the detoxification of xenobiotics. CYPs in the liver and extrahepatic tissues may also activate drugs and exogenous substances to toxic and/or tumorigenic metabolites. Microsomal and mitochondrial CYPs play key roles in the biosynthesis or catabolism of steroid hormones, bile acids, fat-soluble vitamins, fatty acids, and eicosanoids, which underscores the catalytic versatility of cytochrome P₄₅₀ enzymes. For these reasons, increased predictability in CYP activity would be a boon to drug design and treatment.

However, the pharmacologic or toxic effects of certain drugs are exaggerated in a significant percentage of the population due to genetic deficiencies in one or more cytochrome P₄₅₀ enzymes, and this is the key to interpatient variability. The two major polymorphically expressed P₄₅₀ enzymes are CYP2D6 and CYP2C19, although allelic variants have been described for nearly all of the human CYPs involved in drug metabolism. Individuals lacking CYP2D6 or CYP2C19 were initially identified as poor metabolizers of debrisoquine and S-mephenytoin, respectively. However, because each P₄₅₀ enzyme has broad substrate specificity, each genetic defect effects the metabolism of several drugs. The incidence of the poor-metabolizer phenotype varies among different ethnic groups. The observation that individuals who are genetically deficient in a particular P₄₅₀ enzyme are poor metabolizers of one or more drugs illustrates a very important principle - namely that the rate of elimination of drugs can be largely determined by a single P₄₅₀ enzyme. This observation seems to contradict the fact that CYPs have broad and overlapping substrate specificities. The resolution to this apparent paradox lies in the fact that, although more than one human P₄₅₀ enzyme can catalyze the metabolism of a drug, they may do so with markedly different affinities. Consequently, drug metabolism in vivo, where only low substrate concentrations are usually achieved, is often determined by the P₄₅₀ enzyme with the highest affinity (lowest apparent Kₘ) for the drug.

This difference in CYP expression and activity amongst human subpopulations also leads to differences in reported "adverse effects," including so-called "side effects," of commonly used medications. It is frequently the case that the utility of a drug for its therapeutic effect is limited by the unintended, adverse effects that accompany the primary and desired activity, in that a patient may not be able to tolerate an efficacious dose. Alternatively, the therapeutic window of a drug may be monitored by a health care provider. Ideally, therapeutic drug monitoring should cost-effectively lead to improved efficacy of the drug and to a reduction in side effects, but this is often not the case. Due to mounting pressures of time and cost in the health care system at large, the burden of monitoring therapeutic drugs which have a high interpatient variability may deter their prescription altogether. Thus, therapeutic drug monitoring is ineffective and inefficient when there is a large interpatient variability. The situation calls for a better solution.

The basic reaction catalyzed by cytochrome P₄₅₀ is monooxygenation which one atom of oxygen is incorporated into a substrate, designated RH, and the other is reduced to water with reducing equivalents derived from NADPH, as follows :

Substrate (RH) + O₂ + NADPH + H⁺ -> Product (ROH) + H₂O + NADP⁺

In the endoplasmic reticulum, where most of the P₄₅₀ enzymes involved in biotransformation are localized, electrons are relayed from NADPH to cytochrome P₄₅₀ via a flavoprotein called NADPH-cytochrome P₄₅₀ reductase. Within this flavoprotein, electrons are transferred from NADPH to cytochrome P₄₅₀ via FMN and FAD. There are some notable exceptions to the general rule that cytochrome P₄₅₀ requires a second enzyme (i.e., a flavoprotein) for catalytic activity. Cytochrome P₄₅₀ catalyzes several types of oxidation reactions, including:
1. Hydroxylation of an aliphatic or aromatic carbon;
2. Epoxidation of a double bond;
3. Heteroatom (*S*-, *N*-, and *I*-) oxygenation and N-hydroxylation;
4. Heteroatom *(O*-, *S*-, *N*- and *Si*-) dealkylation;
5. Oxidative group transfer;
6. Cleavage of esters; and
7. Dehydrogenation.

The hydroxylation of may proceed via an oxirane intermediate (i.e., an arene oxide) that isomerizes to the corresponding phenol. Alternatively aromatic hydroxylation can proceed by a mechanism known as direct insertion. For example, the *ortho*-hydroxylation and *para*-hydroxylation of chlorobenzene proceed via 2,3- and 3,4-epoxidation, whereas *meta*-hydroxylation proceeds by direct insertion. When hydroxylation involves direct insertion, hydrogen abstraction (i.e., cleavage of the C-H bond) is the rate-limiting step, so that substitution of hydrogen with deuterium considerably slows the hydroxylation reaction. This phenomenon is known as the Deuterium Kinetic Isotope Effect (DKIE).

The bond strength is directly proportional to the absolute value of the ground-state vibrational energy of the bond. This vibrational energy, which is also known as the zero-point vibrational energy, depends on the mass of the atoms that form the bond. The absolute value of the zero-point vibrational energy increases as the mass of one or both of the atoms making the bond increases. Since deuterium (D) is two-fold more massive than hydrogen (H), it follows that a C-D bond is stronger than the corresponding C-H bond. Compounds with C-D bonds are frequently indefinitely stable in H₂O, and have been widely used for isotopic studies. If a C-H bond is broken during a rate-determining step in a chemical reaction (i.e. the step with the highest transition state energy), then substituting a deuterium for that hydrogen will cause a decrease in the reaction rate and the process will slow down.

The deuterium kinetic isotope effect (DKIE) can range from about 1 (no isotope effect) to very large numbers, such as 50 or more, meaning that the reaction can be fifty, or more, times slower when deuterium is substituted for hydrogen. High DKIE values may be due in part to a phenomenon known as tunneling, which is a consequence of the uncertainty principle. Tunneling is ascribed to the small size of a hydrogen atom, and occurs because transition states involving a proton can sometimes form in the absence of the required activation energy. A deuterium is larger and statistically has a much lower probability of undergoing this phenomenon. Substitution of tritium for hydrogen results in yet a stronger bond than deuterium and gives numerically larger isotope effects. Tritium, however, is radioactive, and therefore not suited for incorporation into pharmaceuticals.

Discovered in 1932 by Urey, deuterium (D) is a stable and nonradioactive isotope of hydrogen. It was the first isotope to be separated from its element in pure form and is twice as massive as hydrogen, and makes up about 0.02% of the total mass of hydrogen (in this usage meaning all hydrogen isotopes) on earth. When two deuteriums bind to one oxygen, deuterium oxide (D₂O or "heavy water") is formed. D₂O looks and tastes like H₂O but it has different physical properties. It boils at 101.41 °C and freezes at 3.79 °C. Its heat capacity, heat of fusion, heat of vaporization, and entropy are all higher than H₂O. It is also more viscous and is not as powerful a solvent as H₂O.

Deuteration of pharmaceuticals to improve pharmacokinetics (PK), pharmacodynamics (PD), and toxicity profiles, has been demonstrated previously with some classes of drugs. For example, DKIE was used to decrease the hepatotoxicity of halothane by presumably limiting the production of reactive species such as trifluoroacetyl chloride. However, this method may not be applicable to all drug classes. For example, deuterium incorporation can lead to metabolic switching which may even give rise to an oxidative intermediate with a faster off-rate from an activating Phase I enzyme (e.g. CYP3A4). The concept of metabolic switching asserts that xenogens, when sequestered by Phase I enzymes, may bind transiently and re-bind in a variety of conformations prior to the chemical reaction (e.g. oxidation). This claim is supported by the relatively vast size of binding pockets in many phase I enzymes and the promiscuous nature of many metabolic reactions. Metabolic switching can potentially lead to different proportions of known metabolites as well as altogether new metabolites. This new metabolic profile may impart more or less toxicity. Such pitfalls are non-obvious and have not been heretofore sufficiently predictable *a priori* for any drug class.

Efforts to modify the metabolic parameter via other means have also been pursued, of course; along with the crafting of more potent and efficacious molecules, this is the aim of the disciplines of medicinal chemistry and rational drug design. Thus, metabolically labile esters and ethers may be replaced, lipophilicity or aqueous solubility altered by the substitution or polar or non-polar substituents, molecular geometry altered to better fit a binding site on a target protein, and so forth. One could conceive of using traditional methods to design a molecule that lacked known offending CYP metabolic sites. However, corresponding difficulties arise when polar regions of molecules, whose adjacent hydrogen-bearing atoms are susceptible to metabolism by CYPs, are eliminated, as these very regions often give the molecule its ligand-binding and other useful properties. Deuteration presents a unique solution in that it permits the design of pharmaceuticals to proceed, if not uninhibited, then less inhibited by concerns about metabolic liability. More importantly, it provides a solution to the problem of disfavored, metabolically labile drugs which, due to their variable effects, would otherwise have wider acceptance and utility. Finally, deuteration presents a way to improve even those drugs which already enjoy wide acceptance due to the critical nature of their primary effect, but which cause patients reduced quality of life due to adverse side effects.

Accordingly, provided herein is a method of reducing inter-patient variability in a drug treatment, comprising;
i. identifying inter-patient variability in said drug treatment;
ii. preparing a deuterated analog of said drug; and
iii. using said deuterated analog in said drug treatment to reduce inter-patient variability of said drug treatment.

In certain embodiments, said deuterated analog has deuteration at one or more sites metabolized by a cytochrome P₄₅₀. In further embodiments, said cytochrome P450 is a polymorphically expressed cytochrome P₄₅₀. In yet further embodiments, said polymorphically expressed cytochrome P₄₅₀ is selected from the group consisting of CYP3A4, CYP2D6 and CYP2C19.

Also provided is a method of reducing interpatient variability in a population of patients taking a drug comprising
i. identifying a population of patients having interpatient variability; and
ii. administering to a population of patients a deuterated analogue of a drug.

In certain embodiments, said deuterated analogue has deuteration at one or more sites metabolized by a cytochrome P₄₅₀. In further embodiments, said cytochrome P450 is a polymorphically expressed cytochrome P₄₅₀. In yet further embodiments, said polymorphically expressed cytochrome P₄₅₀ is selected from the group consisting of CYP3A4, CYP2D6 and CYP2C19.

In certain embodiments, said interpatient variability is in the frequency or severity of side effects experienced. In other embodiments, said interpatient variability is in the half-life (t_{½}) of a drug in the plasma. In other embodiments, said interpatient variability is in the maximum plasma concentration (Cₘₐₓ) of a drug in the plasma. In other embodiments, said interpatient variability is in the time at maximal plasma concentration (Tₘₐₓ) of a drug in the plasma. In other embodiments, said interpatient variability is in the area under the time-versus-concentration curve (AUC) of a drug in the plasma. In other embodiments, said interpatient variability is in the metabolite profile of the drug.

In certain embodiments, said drug is venlafaxine.

In certain embodiments, using said deuterated analog yields a reduced inter-patient variability in plasma half life (t½) of 20% or more compared to non-deuterated venlafaxine.

In certain embodiments, using said deuterated analog yields a reduced inter-patient variability in maximum plasma concentration (Cₘₐₓ) of 30% or more compared to non-deuterated venlafaxine.

In certain embodiments, using said deuterated analog yields a reduced inter-patient variability in time at maximal plasma concentration (Tₘₐₓ) of 15% or more compared to non-deuterated venlafaxine.

In certain embodiments, using said deuterated analog yields a reduced inter-patient variability in plasma area under the time-versus-concentration curve (AUC) of 20% or more compared to non-deuterated venlafaxine.

In certain embodiments, using said deuterated analog yields a reduction in the incidence of one or more side effects.

In certain embodiments, using said deuterated analog yields at least 25% fewer side effects overall than non-deuterated venlafaxine.

In certain embodiments, using said deuterated analog yields at least 30% fewer side effects overall than non-deuterated venlafaxine.

In certain embodiments, using said deuterated analog yields at least 35% fewer side effects overall than non-deuterated venlafaxine.

In certain embodiments, the side effect is selected from the group consisting of drowsiness, dry mouth, fatigue or somnolence, headache, lightheadedness, nausea, emesis, mydriasis, anxiety, nervousness, and insomnia, dizziness, anxiety, asthenia, sweating, anorexia, weight gain, activation of mania/hypomania, and suicidal ideation. In other embodiments, the side effect is selected from the group consisting of hyponatremia and/or the syndrome of inappropriate antidiuretic hormone secretion (SIADH), seizures, abnormal bleeding (most commonly ecchymosis), increases in serum cholesterol, interstitial lung disease, eosinophilic pneumonia, gastrointestinal, and urogenital disorders.

Also provided is a deuterated analogue of a drug having reduced interpatient variability.

In certain embodiments, the drug is venlafaxine. Accordingly, also provided is a deuterated analogue of venlafaxine having a reduced inter-patient variability in plasma half life of 20% or more compared to non-deuterated venlafaxine. Also provided is a deuterated analogue of venlafaxine having a reduced inter-patient variability in maximum plasma concentration in humans of 30% or more compared to non-deuterated venlafaxine. Also provided is a deuterated analogue of venlafaxine having a reduced inter-patient variability time at maximal plasma concentration in humans of 15% or more compared to non-deuterated venlafaxine. Also provided is a deuterated analogue of venlafaxine having a reduced inter-patient variability in plasma area under the time-versus-concentration curve 20% or more compared to non-deuterated venlafaxine. Also provided is a deuterated analogue of venlafaxine which causes at least 25% fewer side effects than non-deuterated venlafaxine.

It has been hypothesized that the efficacy of venlafaxine (Effexor^{®}) is mainly due to its ability to inhibit serotonin reuptake and, potentially, norepinephrine reuptake in neuronal cells. The latter is purported to take effect only at high doses. The drug substance is sold as a 50/50 racemic mixture of R- and S-enantiomers. The mechanism of action of this drug has been extensively studied.

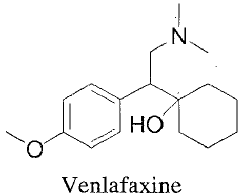

The benefits and shortcomings of this drug have been extensively reviewed as well. Some of these shortcomings can be traced to metabolism-related phenomena. Venlafaxine is converted *in vivo* by oxidative and conjugative degradation to multiple metabolites, at least 48 of which are documented. The major metabolites include much phase I metabolism leading to demethylation at the oxygen and/or nitrogen centers, and cyclohexyl ring hydroxylation, as well as significant phase II metabolism including glucuronidation of the hydroxylated metabolites. Because this drug is metabolized by polymorphically-expressed isozymes of cytochrome P₄₅₀ including CYPs 2C19 and 2D6, and because it can act as an inhibitor of CYP2D6, its application in polypharmacy is necessarily complex and has potential for adverse events. These CYPs are involved in the metabolism of many medications that are typically prescribed concurrently with venlafaxine. This phenomenon increases inter-patient variability in response to polypharmacy. An example of the critical need for improvement is the published interpatient variability observed in "poor metabolizers" having either defective CYP2D6 alleles or total lack of CYP2D6 expression. These patients fail to convert venlafaxine to its equipotent metabolite, O-desmethylvenlafaxine. Venlafaxine also suffers from a short half-life relative to the majority of serotonin reuptake inhibitors. The half-life of venlafaxine in humans is ~5 hours, while its active metabolite has a T_{1/2} of ~11 hours. As a consequence of its 5 - 11 hour pharmacological half-life, those taking venlafaxine are at significant risk of SRI discontinuation symptoms if the drug is abruptly discontinued. Furthermore, in order to overcome its short half-life, the drug must be taken 2 (BID) or 3 (TID) times a day, which increases the probability of patient incompliance and discontinuance. Most other serotonin reuptake inhibitors (SRIs) have half-lives ≥ 24 hours. A 24-72 hour half-life is regarded as ideal for this class of compounds by most clinicians. There is therefore an obvious and immediate need for improvements in the development of monoamine reuptake inhibitors such as paroxetine.

The carbon-hydrogen bonds of venlafaxine contain a naturally occurring distribution of hydrogen isotopes, namely ¹H or protium (about 99.9844%), ²H or deuterium (about 0.0156%), and ³H or tritium (in the range between about 0.5 and 67 tritium atoms per 10¹⁸ protium atoms). Increased levels of deuterium incorporation produce a detectable Kinetic Isotope Effect (KIE) that could affect the pharmacokinetic, pharmacologic and/or toxicologic parameters of such monoamine reuptake inhibitors relative to compounds having naturally occurring levels of deuterium. Disclosed herein is a novel approach to designing and synthesizing new analogs of drugs such as venlafaxine through chemical modifications and derivations of the carbon-hydrogen bonds of the modulators and/or of the chemical precursors used to synthesize said modulators. Suitable modifications of certain carbon-hydrogen bonds into carbon-deuterium bonds may generate novel monoamine reuptake inhibitors with unexpected and non-obvious improvements of pharmacological, pharmacokinetic and toxicological properties in comparison to the non-isotopically enriched monoamine reuptake inhibitors. Judicious and successful application of chemical kinetics to drug design is a key component of this design process. Deuterium incorporation levels in the deuterated analogues disclosed herein are significantly higher than the naturally-occurring levels and are sufficient to induce at least one substantial improvement as described herein.

Judicious substitution with deuterium is useful in solving the pharmacokinetic shortcomings for venlafaxine. For example, both N-methyl groups, the single O-methyl, and several sites on the cyclohexyl ring of venlafaxine are now known to be sites of cytochrome P₄₅₀ metabolism. The toxicities of all resultant metabolites are not known. Furthermore, because polymorphically expressed CYPs such as 2C19 and 2D6 oxidize venlafaxine, and because venlafaxine inhibits the polymorphically expressed CYP2D6, the prevention of such interactions decreases interpatient variability, decreases drug-drug interactions, increases T_{1/2}, decreases the necessary Cₘₐₓ, and improves several other pharmacokinetic parameters. For example, the half-life of the parent drug of venlafaxine ranges from 3 - 7 hours. The equipotent metabolite, O-demethylated venlafaxine, has a half-life averaging 11 hours. Various deuteration patterns can be used to a) alter the ratio of active metabolites, b) reduce or eliminate unwanted metabolites, c) increase the half-life of the parent drug, and /or d) increase the half-life of active metabolites and create a more effective drug and a safer drug for polypharmacy, whether the polypharmacy be intentional or not. High doses of venalxafine are often prescribed in order to reach levels capable of inhibiting norepinephrine reuptake. Unfortunately, high doses are also associated with hypertension. Since these phenomena are linked by the pharmaceutical agent rather than the pharmacological target, the two phenomena are theoretically separable by increasing the half-life thus allowing dosing in a range that lowers the Cₘₐₓ and thus may avoid triggering the mechanism leading to hypertension. Further illustrating this point, venlafaxine is known to display linear kinetics at the low end of the dose range, 75 mg/day, but displays non-linear kinetics at the high end of the dose range, ~400 mg/day, as a result of the saturation of clearance mechanisms. This nonlinearity produces an ascending, rather than a flat, dose-response curve for venlafaxine. The deuteration approach has strong potential to slow metabolism through the previously saturated mechanism allowing linear, more predictable ADMET responses throughout the dose range (which would also be lower). This leads to lesser interpatient variability of the type that can lead to the hypertensive effects.

The deuterated analogs disclosed herein have been demonstrated to maintain the beneficial aspects of the non-isotopically enriched drugs while increasing the half-life (T_{1/2}), lowering the maximum plasma concentration (Cₘₐₓ) of the minimum efficacious dose (MED), lowering the efficacious dose and thus decreasing the non-mechanism-related toxicity, and/or lowering the probability of drug-drug interactions. These drugs also have strong potential to reduce the cost-of-goods (COG) owing to the ready availability of inexpensive sources of deuterated reagents combined with previously mentioned potential for lowering the therapeutic dose.

Deuterated analogs of venlafaxine have the generic Formula 1: 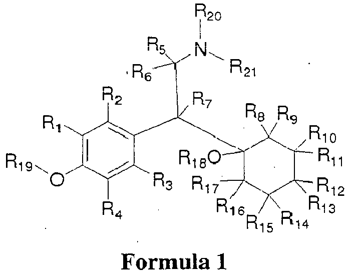 or a pharmaceutically acceptable salt, solvate, or prodrug thereof, wherein:
R₁, R₂, R₃, R₄, R₅, R₆, R₇, R₈, R₉, R₁₀, R₁₁, R₁₂, R₁₃, R₁₄, R₁₅, R₁₆, R₁₇, and R₁₈ are independently selected from the group consisting of hydrogen, and deuterium;

R₁₉, R₂₀, and R₂₁ are independently selected from the group consisting of-CH₃, -CH₂D, -CHD₂, and -CD₃;
provided that compounds of Formula 1 contain at least one deuterium atom; and provided that deuterium enrichment in compounds of Formula 1 is at least about 1%.

Also disclosed herein are pharmaceutical compositions comprising a compound of Formula 1, a single enantiomer of a compound of Formula 1, a mixture of the (+)-enantiomer and the (-)-enantiomer, a mixture of about 90% or more by weight of the (-)-enantiomer and about 10% or less by weight of the (+)-enantiomer, a mixture of about 90% or more by weight of the (+)-enantiomer and about 10% or less by weight of the (-)-enantiomer, an individual diastereomer of a compound of Formula 1, a mixture of diastereomers, or a pharmaceutically acceptable salt, solvate, or prodrug thereof, with a pharmaceutically acceptable carrier.

In further embodiments, R₁₉ is -CD₃. In other embodiments, R₂₀ is-CD₃. In still other embodiments, R₂₁ is -CD₃.

In further embodiments, R₁₉ is not -CH₃. In other embodiments, R₂₀ is not -CH₃. In still other embodiments, R₂₁ is not -CH₃.

In further embodiments, R₁₉ is not -CD₃. In other embodiments, R₂₀ is not
-CD₃. In still other embodiments, R₂₁ is not -CD₃.

In further embodiments, R₁₉ is -CH₃. In other embodiments, R₂₀ is-CH₃. In still other embodiments, R₂₁ is -CH₃.

In certain embodiments, the compound is :

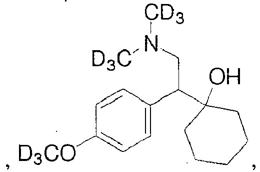

In certain embodiments, the compound of Formula 1 contains about 60% or more by weight of the (-)-enantiomer of the compound and about 40% or less by weight of (+)-enantiomer of the compound. In some embodiments, the compound of Formula 1 contains about 70% or more by weight of the (-)-enantiomer of the compound and about 30% or less by weight of (+)-enantiomer of the compound. In some embodiments, the compound of Formula 1 contains about 80% or more by weight of the (-)-enantiomer of the compound and about 20% or less by weight of (+)-enantiomer of the compound. In some embodiments, the compound of Formula 1 contains about 90% or more by weight of the (-)-enantiomer of the compound and about 10% or less by weight of the (+)-enantiomer of the compound. In some embodiments, the compound of Formula 1 contains about 95% or more by weight of the (-)-enantiomer of the compound and about 5% or less by weight of (+)-enantiomer of the compound. In some embodiments, the compound of Formula 1 contains about 99% or more by weight of the (-)-enantiomer of the compound and about 1 % or less by weight of (+)-enantiomer of the compound.

In certain other embodiments, the compound of Formula 1 contains about 60% or more by weight of the (+)-enantiomer of the compound and about 40% or less by weight of (-)-enantiomer of the compound. In some embodiments, the compound of Formula 1 contains about 70% or more by weight of the (+)-enantiomer of the compound and about 30% or less by weight of (-)-enantiomer of the compound. In some embodiments, the compound of Formula 1 contains about 80% or more by weight of the (+)-enantiomer of the compound and about 20% or less by weight of (-)-enantiomer of the compound. In some embodiments, the compound of Formula 1 contains about 90% or more by weight of the (+)-enantiomer of the compound and about 10% or less by weight of the (-)-enantiomer of the compound. In some embodiments, the compound of Formula 1 contains about 95% or more by weight of the (+)-enantiomer of the compound and about 5% or less by weight of (-)-enantiomer of the compound. In some embodiments, the compound of Formula 1 contains about 99% or more by weight of the (+)-enantiomer of the compound and about 1% or less by weight of (-)-enantiomer of the compound.

Further, disclosed herein are methods of eliciting, modulating and/or regulating the reuptake of monoamine neurotransmitters including serotonin and/or norepinephrine.

In some embodiments, the inter-patient variation in plasma levels of the compounds, or metabolites thereof, is decreased by greater than about 10%, as compared to the non-isotopically enriched compounds. In further embodiments, the inter-patient variation in plasma levels of the compounds, or metabolites thereof, is decreased by greater than about 15%, as compared to the non-isotopically enriched compounds. In further embodiments, the inter-patient variation in plasma levels of the compounds, or metabolites thereof, is decreased by greater than about 20%, as compared to the non-isotopically enriched compounds. In further embodiments, the inter-patient variation in plasma levels of the compounds, or metabolites thereof, is decreased by greater than about 25%, as compared to the non-isotopically enriched compounds. In further embodiments, the inter-patient variation in plasma levels of the compounds, or metabolites thereof, is decreased by greater than about 30%, as compared to the non-isotopically enriched compounds. In further embodiments, the inter-patient variation in plasma levels of the compounds, or metabolites thereof, is decreased by greater than about 40%, as compared to the non-isotopically enriched compounds. In further embodiments, the inter-patient variation in plasma levels of the compounds, or metabolites thereof, is decreased by greater than about 45%, as compared to the non-isotopically enriched compounds. In further embodiments, the inter-patient variation in plasma levels of the compounds, or metabolites thereof, is decreased by greater than about 50%, as compared to the non-isotopically enriched compounds. Plasma levels of the compounds, or metabolites thereof, may be measured by the methods of Li et al Rapid Communications in Mass Spectrometry 2005, 19(14), 1943-1950, which is hereby incorporated by reference in its entirety.

In some embodiments, the inter-patient variation in plasma half-life of the compounds, or metabolites thereof, is decreased by greater than about 10%, as compared to the non-isotopically enriched compounds. In further embodiments, the inter-patient variation in plasma half-life of the compounds, or metabolites thereof, is decreased by greater than about 15%, as compared to the non-isotopically enriched compounds. In further embodiments, the inter-patient variation in plasma half-life of the compounds, or metabolites thereof, is decreased by greater than about 20%, as compared to the non-isotopically enriched compounds. In further embodiments, the inter-patient variation in plasma half-life of the compounds, or metabolites thereof, is decreased by greater than about 25%, as compared to the non-isotopically enriched compounds. In further embodiments, the inter-patient variation in plasma half-life of the compounds, or metabolites thereof, is decreased by greater than about 30%, as compared to the non-isotopically enriched compounds. In further embodiments, the inter-patient variation in plasma half-life of the compounds, or metabolites thereof, is decreased by greater than about 40%, as compared to the non-isotopically enriched compounds. In further embodiments, the inter-patient variation in plasma half-life of the compounds, or metabolites thereof, is decreased by greater than about 45%, as compared to the non-isotopically enriched compounds. In further embodiments, the inter-patient variation in plasma half-life of the compounds, or metabolites thereof, is decreased by greater than about 50%, as compared to the non-isotopically enriched compounds.

In some embodiments, the inter-patient variation in time at maximum plasma concentration of the compounds, or metabolites thereof, is decreased by greater than about 10%, as compared to the non-isotopically enriched compounds. In further embodiments, the inter-patient variation in time at maximum plasma concentration of the compounds, or metabolites thereof, is decreased by greater than about 15%, as compared to the non-isotopically enriched compounds. In further embodiments, the inter-patient variation in time at maximum plasma concentration of the compounds, or metabolites thereof, is decreased by greater than about 20%, as compared to the non-isotopically enriched compounds. In further embodiments, the inter-patient variation in time at maximum plasma concentration of the compounds, or metabolites thereof, is decreased by greater than about 25%, as compared to the non-isotopically enriched compounds. In further embodiments, the inter-patient variation in time at maximum plasma concentration of the compounds, or metabolites thereof, is decreased by greater than about 30%, as compared to the non-isotopically enriched compounds. In further embodiments, the inter-patient variation in time at maximum plasma concentration of the compounds, or metabolites thereof, is decreased by greater than about 40%, as compared to the non-isotopically enriched compounds. In further embodiments, the inter-patient variation in time at maximum plasma concentration of the compounds, or metabolites thereof, is decreased by greater than about 45%, as compared to the non-isotopically enriched compounds. In further embodiments, the inter-patient variation in time at maximum plasma concentration of the compounds, or metabolites thereof, is decreased by greater than about 50%, as compared to the non-isotopically enriched compounds.

In some embodiments, the inter-patient variation in maximum plasma concentration of the compounds, or metabolites thereof, is decreased by greater than about 10%, as compared to the non-isotopically enriched compounds. In further embodiments, the inter-patient variation in maximum plasma concentration of the compounds, or metabolites thereof, is decreased by greater than about 15%, as compared to the non-isotopically enriched compounds. In further embodiments, the inter-patient variation in maximum plasma concentration of the compounds, or metabolites thereof, is decreased by greater than about 20%, as compared to the non-isotopically enriched compounds. In further embodiments, the inter-patient variation in maximum plasma concentration of the compounds, or metabolites thereof, is decreased by greater than about 25%, as compared to the non-isotopically enriched compounds. In further embodiments, the inter-patient variation in maximum plasma concentration of the compounds, or metabolites thereof, is decreased by greater than about 30%, as compared to the non-isotopically enriched compounds. In further embodiments, the inter-patient variation in maximum plasma concentration of the compounds, or metabolites thereof, is decreased by greater than about 40%, as compared to the non-isotopically enriched compounds. In further embodiments, the inter-patient variation in maximum plasma concentration of the compounds, or metabolites thereof, is decreased by greater than about 45%, as compared to the non-isotopically enriched compounds. In further embodiments, the inter-patient variation in maximum plasma concentration of the compounds, or metabolites thereof, is decreased by greater than about 50%, as compared to the non-isotopically enriched compounds.

In some embodiments, the inter-patient variation in area under the time vs. concentration curve of the compounds, or metabolites thereof, is decreased by greater than about 10%, as compared to the non-isotopically enriched compounds. In further embodiments, the inter-patient variation in area under the time vs. concentration curve of the compounds, or metabolites thereof, is decreased by greater than about 15%, as compared to the non-isotopically enriched compounds. In further embodiments, the inter-patient variation in area under the time vs. concentration curve of the compounds, or metabolites thereof, is decreased by greater than about 20%, as compared to the non-isotopically enriched compounds. In further embodiments, the inter-patient variation in area under the time vs. concentration curve of the compounds, or metabolites thereof, is decreased by greater than about 25%, as compared to the non-isotopically enriched compounds. In further embodiments, the inter-patient variation in area under the time vs. concentration curve of the compounds, or metabolites thereof, is decreased by greater than about 30%, as compared to the non-isotopically enriched compounds. In further embodiments, the inter-patient variation in area under the time vs. concentration curve of the compounds, or metabolites thereof, is decreased by greater than about 40%, as compared to the non-isotopically enriched compounds. In further embodiments, the inter-patient variation in area under the time vs. concentration curve of the compounds, or metabolites thereof, is decreased by greater than about 45%, as compared to the non-isotopically enriched compounds. In further embodiments, the inter-patient variation in area under the time vs. concentration curve of the compounds, or metabolites thereof, is decreased by greater than about 50%, as compared to the non-isotopically enriched compounds.

In certain embodiments are provided methods of reducing inter-patient variability in 1) the rate of a reaction catalyzed by, or 2) the quantity of at least one metabolite produced by, at least one cytochrome P₄₅₀ isoform in mammalian subjects as compared to the non-isotopically enriched compound. Examples of cytochrome P₄₅₀ isoforms in mammalian subjects include CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3ASP1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51 and the like.

In another embodiment, there are provided methods for treating a mammalian subject, particularly a human, suspected of having, or being prone to a disease or condition involving monoamine reuptake, comprising administering to a mammalian subject in need thereof a therapeutically effective amount of a monoamine reuptake inhibitor comprising at least one of the compounds of Formula 1 biogenic monoamine levels as compared to the non-isotopically enriched compound.

In another aspect, there are provided methods for treating a mammalian subject, particularly a human, suspected of having, or being prone to a disease or condition involving monoamine reuptake, comprising administering to a mammalian subject in need thereof a therapeutically effective amount of a monoamine reuptake inhibitor comprising at least one of the compounds of Formula 1. Examples of improved clnical effects include but are not limited to accelerated rate of healing, accelerated rate of symptom relief, improved patient compliance, and/or reduced substance abuse withdrawal symptomology during the treatment.

In another aspect, there are provided methods for treating a mammalian subject, particularly a human, suspected of having, or being prone to a disease or condition involving monoamine reuptake, comprising administering to a mammalian subject in need thereof a therapeutically effective amount of a monoamine reuptake inhibitor comprising at least one of the compounds of Formula 1.

In some embodiments, disease or condition involving monoamine reuptake is selected from the group consisting of anxiety disorder, generalized anxiety disorder, depression, post-traumatic stress disorder, obsessive-compulsive disorder, panic disorder, hot flashes, senile dementia, migraine, hepatopulmonary syndrome, chronic pain, nociceptive pain, neuropathic pain, painful diabetic retinopathy, bipolar depression, obstructive sleep apnea, psychiatric disorders, premenstrual dysphoric disorder, social phobia, social anxiety disorder, urinary incontinence, anorexia, bulimia nervosa, obesity, ischemia, head injury, calcium overload in brain cells, drug dependence, and premature ejaculation.

As used herein, the terms below have the meanings indicated.

When ranges of values are disclosed, and the notation "from n₁ ... to n₂" or "between n₁ ... and n₂" is used, where n₁ and n₂ are the numbers, then unless otherwise specified, this notation is intended to include the numbers themselves and the range between them. This range may be integral or continuous between and including the end values. By way of example, the range "from 2 to 6 carbons" is intended to include two, three, four, five, and six carbons, since carbons come in integer units. Compare, by way of example, the range "from 1 to 3 µM (micromolar)," which is intended to include 1 µM, 3 µM, and everything in between to any number of significant figures (e.g., 1.255 µM, 2.1 µM, 2.9999 µM, etc.).

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

"Deuterium enrichment" refers to the percentage of incorporation of deuterium at a given site on the molecule instead of a hydrogen atom. For example, deuterium enrichment of 1% means that in 1% of molecules in a given sample a particular site is occupied by deuterium. Because the naturally occurring distribution of deuterium is about 0.0156%, deuterium enrichment in compounds synthesized using non-enriched starting materials is about 0.0156%. In some embodiments, the deuterium enrichment in the compounds disclosed herein is greater than 10%. In other embodiments, the deuterium enrichment in the compounds disclosed herein is greater than 20%. In further embodiments, the deuterium enrichment in the compounds disclosed herein is greater than 50%. In some embodiments, the deuterium enrichment in the compounds disclosed herein is greater than 70%. In some embodiments, the deuterium enrichment in the compounds disclosed herein is greater than 90%.

"Isotopic enrichment" refers to the percentage of incorporation of a less prevalent isotope of an element at a given site on the molecule instead of the more prevalent isotope of the element. "Non-isotopically enriched" refers to a molecule in which the percentage of the various isotopes is substantially the same as the naturally occurring percentages.

Included within the scope of compounds disclosed herein are all isotopes of all atoms occurring in the present compounds. Isotopes include those atoms having the same atomic number but different mass numbers. By way of general example and without limitation, isotopes of hydrogen include deuterium (D) and tritium (T). Isotopes of carbon include ¹³C and ¹⁴C. Isotopes of sulfur include ³²S, ³³S ³⁴S and ³⁶S. Isotopes of nitrogen include ¹⁴N and ¹⁵N. Isotopes of oxygen include ¹⁶O, ¹⁷O, and ¹⁸O.

Isotopic hydrogen can be introduced into organic molecules by synthetic techniques that employ deuterated reagents whereby incorporation rates are predetermined and/or by exchange techniques wherein incorporation rates are determined by equilibrium conditions and may be highly variable depending on the reaction conditions. Synthetic techniques, where tritium or deuterium is directly and specifically inserted by tritiated or deuterated reagents of known isotopic content, may yield high tritium or deuterium abundance, but can be limited by the chemistry required. In addition, the molecule being labeled may be changed, depending upon the severity of the synthetic reaction employed. Exchange techniques, on the other hand, may yield lower tritium or deuterium incorporation, often with the isotope being distributed over many sites on the molecule, but offer the advantage that they do not require separate synthetic steps and are less likely to disrupt the structure of the molecule being labeled.

The verb "deuterated," and its conjugations such as "deuterating," means to synthesize a compound in which one or more deuterium atoms take the place of one ot more hydrogens. The deuterating process can be carried out by replacing hydrogens on an otherwise fully synthesized drug or target end product molecule, but more often deuterating is carried out via incorporation of deuterium into reagents which will become part of the deuterated drug or deuterated molecule.

In some embodiments, the administering step in the methods disclosed herein comprises administering the compound in some composition, such as for example a single tablet, pill, capsule, a single solution for intravenous injection, a single drinkable solution, a single dragee formulation or patch, and the like wherein the amount administered is about 0.5 milligram to 400 milligram total daily dose.

The term "pharmaceutically acceptable" refers to a compound, additive or composition that is not biologically or otherwise undesirable. For example, the additive or composition may be administered to a subject along with a compound without causing any undesirable biological effects or interacting in an undesirable manner with any of the other components of the pharmaceutical composition in which it is contained.

The term "pharmaceutically acceptable salts" includes hydrochloric salt, hydrobromic salt, hydroiodic salt, hydrofluoric salt, sulfuric salt, citric salt, maleic salt, acetic salt, lactic salt, nicotinic salt, succinic salt, oxalic salt, phosphoric salt, malonic salt, salicylic salt, phenylacetic salt, stearic salt, pyridine salt, ammonium salt, piperazine salt, diethylamine salt, nicotinamide salt, formic salt, urea salt, sodium salt, potassium salt, calcium salt, magnesium salt, zinc salt, lithium salt, cinnamic salt, methylamino salt, methanesulfonic salt, picric salt, tartaric salt, triethylamino salt, dimethylamino salt, tris(hydroxymethyl)aminomethane salt and the like. Additional pharmaceutically acceptable salts are known to those of skill in the art.

When used in conjunction with a compound of this invention, the terms "elicit", "eliciting," "modulator", "modulate", "modulating", "regulator", "regulate" or "regulating" the activity refer to a compound that can act as an agonist, an inverse agonist, an inhibitor, or an antagonist of a particular enzyme or receptor, such as for example a serotonin receptor.

The terms "drug", "therapeutic agent" and "chemotherapeutic agent", refer to a compound or compounds and pharmaceutically acceptable compositions thereof that are administered to mammalian subjects as prophylactic or remedy in the treatment of a disease or medical condition. Such compounds may be administered to the subject via oral formulation, inhalation, intravenous infusion, ocular application, transdermal formulation or by injection.

The term "subject" refers to an animal, preferably a mammal, and most preferably a human, who is the object of treatment, observation or experiment. The mammal may be selected from the group consisting of mice, rats, hamsters, gerbils, rabbits, guinea pigs, dogs, cats, sheep, goats, cows, horses, giraffes, platypuses, primates, such as monkeys, chimpanzees, and apes, and humans.

The term "effective amount" of a compound refers a sufficient amount of the compound that provides a desired effect but with no- or acceptable- toxicity. This amount may vary from subject to subject, depending on the species, age, and physical condition of the subject, the severity of the disease that is being treated, the particular compound used, its mode of administration, and the like. A suitable effective amount may be determined by one of ordinary skill in the art.

The term "therapeutically effective amount" is used to indicate an amount of an active compound, or pharmaceutical agent, that elicits the biological or medicinal response indicated. This response may occur in a tissue, system (animal including human) that is being sought by a researcher, veterinarian, medical doctor or other clinician.

The term "prodrug" refers to an agent that is converted into the parent drug in vivo. Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. A prodrug may be converted into the parent drug by various mechanisms, including enzymatic processes and metabolic hydrolysis.

In light of the purposes described for the present invention, all references to reagents ordinarily containing hydrogens, hydrides, or protons may include partially or fully deuterated versions (containing deuterium, deuteride, or deuteronium) as required to affect transformation to the improved drug substances outlined herein.

The desired dose is preferably presented in the form of one, two, three, four, five, six or more sub-doses that are administered at appropriate intervals per day. The dose or sub-doses can be administered in the form of dosage units containing for instance from 0.5 to 1500 milligram, preferably from 0.5 to 200 milligram and most preferably from 0.5 to 75 milligram active constituent per dosage unit, and if the condition of the patient requires the dose can, by way of alternative, be administered as a continuous infusion.

As used herein, all references to reducing/reduced or decreasing/decreased inter-patient variability are in comparison to the corresponding non-isotopically-enriched or nondeuterated compound.

As used herein, and unless otherwise indicated, the following abbreviations have the following meanings: Me refers to methyl, Et refers to ethyl, i-Pr refers to isopropyl, t-Bu or tert-butyl refers to tertiary butyl, Ph refers to phenyl, Bn refers to benzyl (PhCH₂-), Bz refers to benzoyl (PhCO-), MOM refers to methoxymethyl, Ac refers to acetyl, TMS refers to trimethylsilyl, TBS refers to tert-butyldimethylsilyl, Ms refers to methanesulfonyl (CH₃SO₂-), Ts refers to p-toluenesulfonyl (p-CH₃PhSO₂-), Tf refers to trifluoromethanesulfonyl (CF₃SO₂-), TfO refers to trifluoromethanesulfonate (CF₃SO₃-), D₂O refers to deuterium oxide, DMF refers to N,N-dimethylformamide, DCM refers to dichloromethane (CH₂Cl₂), THF refers to tetrahydrofuran, EtOAc refers to ethyl acetate, Et₂O refers to diethyl ether, MeCN refers to acetonitrile (CH₃CN), NMP refers to 1-N-methyl-2-pyrrolidinone, DMA refers to N,N-dimethylacetamide, DMSO refers to dimethylsulfoxide, DCC refers to 1,3-dicyclohexyldicarbodiimide, EDCI refers to 1-(3-dimethylaminopropyl)-3-ethylcarbodiimide, Boc refers to tert-butylcarbonyl, Fmoc refers to 9-fluorenylmethoxycarbonyl, TBAF refers to tetrabutylammonium fluoride, TBAI refers to tetrabutylammonium iodide, TMEDA refers to N,N,N,N-tetramethylethylene dianine, Dess-Martin periodinane or Dess Martin reagent refers to 1,1,1-triacetoxy-1,1-dihydro-1,2-benziodoxol-3(1H)-one, DMAP refers to 4-N,N-dimethylaminopyridine, (i-Pr)₂NEt or DIEA or Hunig's base refers to N,N-diethylisopropylamine, DBU refers to 1,8-Diazabicyclo[5.4.0]undec-7-ene, (DHQ)₂AQN refers to dihydroquinine anthraquinone-1,4-diyl diether, (DHQ)₂PHAL refers to dihydroquinine phthalazine-1,4-diyl diether, (DHQ)₂PYR refers to dihydroquinine 2,5-diphenyl-4,6-pyrimidinediyl diether, (DHQD)₂AQN refers to dihydroquinidine anthraquinone-1,4-diyl diether, (DHQD)₂PHAL refers to dihydroquinidine phthalazine-1,4-diyl diether, (DHQD)₂PYR refers to dihydroquinidine 2,5-diphenyl-4,6-pyrimidinediyl diether, LDA refers to lithium diisopropylamide, LiTMP refers to lithium 2,2,6,6-tetramethylpiperdinamide, n-BuLi refers to n-butyl lithium, t-BuLi refers to tert-butyl lithium, IBA refers to 1-hydroxy-1,2-benziodoxol-3(1H)-one 1-oxide, OsO₄ refers to osmium tetroxide, m-CPBA refers to meta-chloroperbenzoic acid, DMD refers to dimethyl dioxirane, PDC refers to pyridinium dichromate, NMO refers to N-methyl morpholine-N-oxide, NaHMDS refers to sodium hexamethyldisilazide, LiHMDS refers to lithium hexamethyldisilazide, HMPA refers to hexamethylphosphoramide, TMSCI refers to trimethylsilyl chloride, TMSCN refers to trimethylsilyl cyanide, TBSCl refers to tert-butyldimethylsilyl chloride, TFA refers to trifluoroacetic acid, TFAA refers to trifluoroacetic anhydride, AcOH refers to acetic acid, Ac₂O refers to acetic anhydride, AcCl refers to acetyl chloride, TsOH refers to p-toluenesulfonic acid, TsCl refers to p-toluenesulfonyl chloride, MBHA refers to 4-methylbenzhydrylamine, BHA refers to benzhydrylamine, ZnCl₂ refers to zinc (II) dichloride, BF₃ refers to boron trifluoride, Y(OTf)₂ refers to yttrium (III) trifluoromethanesulfonate, Cu(BF₄)₂ refers to copper (II) tetrafluoroborate, LAH refers to lithium aluminum hydride (LiAlH₄), LAD refers to lithium aluminum deuteride, NaHCO₃ refers to Sodium bicarbonate, K₂CO₃ refers to Potassium carbonate, NaOH refers to sodium hydroxide, KOH refers to potassium hydroxide, LiOH refers to lithium hydroxide, HCl refers to hydrochloric acid, H₂SO₄ refers to sulfuric acid, MgSO₄ refers to magnesium sulfate, and Na₂SO₄ refers to sodium sulfate. ¹H NMR refers to proton nuclear magnetic resonance, ¹³C NMR refers to carbon-13 nuclear magnetic resonance, NOE refers to nuclear overhauser effect, NOESY refers to nuclear overhauser and exchange spectroscopy, COSY refers to homonuclear correlation spectroscopy, HMQC refers to proton detected heteronuclear multiplet-quantum coherence, HMBC refers to heteronuclear multiple-bond connectivity, s refers to singlet, br s refers to broad singlet, d refers to doublet, br d refers to broad doublet, t refers to triplet, q refers to quartet, dd refers to double doublet, m refers to multiplet, ppm refers to parts per million, IR refers to infrared spectrometry, MS refers to mass spectrometry, HRMS refers to high resolution mass spectrometry, EI refers to electron impact, FAB refers to fast atom bombardment, CI refers to chemical ionization, HPLC refers to high pressure liquid chromatography, TLC refer to thin layer chromatography, R_{f} refers to retention factor, Rₜ refers to retention time, GC refers to gas chromatography, min is minutes, h is hours, rt or RT is room or ambient temperature, g is grams, mg is milligrams, kg is kilograms, L is liters, mL is milliliters, mol is moles and mmol is millimoles.

Compounds may be made by the method outlined in Scheme I below, by methods known in the art, or by following, with modifications where appropriate, the methods of the Examples below. For all of the following examples, standard work-up and purification methods can be utilized and will be obvious to those skilled in the art.

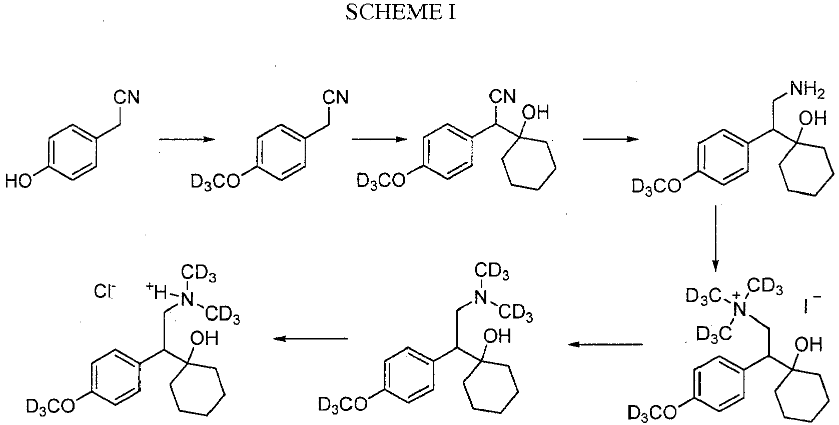

### EXAMPLES

The invention is further illustrated by the following example.

### EXAMPLE 1

### d₉-1-[2-Dimethylamino-1-(4-methoxyphenyl)-ethyl]-cyclohexanol hydrochloride salt

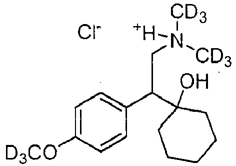

### Step 1

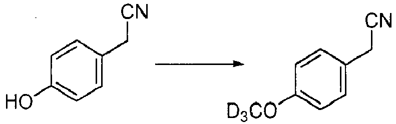

d₃-(4-Methoxyphenyl)-acetonitrile: d₃-Iodomethane (8.70 g, 60 mmol) was added to a stirred solution of (4-hydroxyphenyl)-acetonitrile (4.50 g, 30 mmol) in acetone (30 mL) containing potassium carbonate (6.21 g, 45 mmol) at ambient temperature, and the mixture was heated at reflux overnight, cooled to ambient temperature, filtered, and concentrated to give the crude product, which was purified by flash chromatography using hexanes-ethyl acetate to afford the desired product, d₃-(4-methoxyphenyl)-acetonitrile, as a light yellow oil. Yield: 3.99 g (89%). ¹H-NMR (CDCl₃) δ ppm: 3.67(s, 2H), 6.88(d, 2H, J = 8.7Hz), 7.22(d, 2H, J = 8.7Hz).

### Step 2

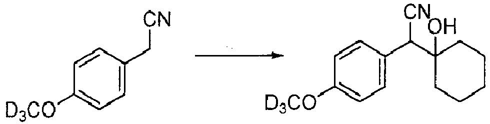

d₃-(1-Hydroxycyclohexyl)-(4-methoxyphenyl)-acetonitrile: Tetra-*n-*butyl ammonium hydrogen sulfate (0.10 g, 0.29 mmol) and 2N NaOH (1.2 mL) were added sequentially to a vigorously stirred d₃-(4-methoxyphenyl)-acetonitrile (0.85 g, 5.66 mmol) at 0°C, and stirring was maintained for 30 minutes. Cyclohexanone (0.67 g, 6.8 mmol) was added to this mixture at 0-5°C over 10 minute. The reaction mixture was allowed to warm to ambient temperature and vigorous stirring was continued for an additional 1 hour. The white precipitate was filtered and washed with water and hexanes to afford the desired product, d₃-(1-hydroxycyclohexyl)-(4-methoxyphenyl)-acetonitrile, as a white solid. Yield: 1.28g (91%). ¹H-NMR (CDCl₃) δ ppm: 1.05-1.80 (m, 10H), 3.73 (s, 1H), 6.90 (d, 2H, J = 8.7Hz), 7.27 (d, 2H, J = 8.7Hz)..

### Step 3

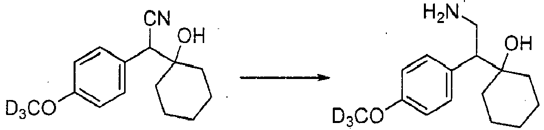

d₃-1-[2-Amino-1-(4-methoxyphenyl)-ethyl]-cyclohexanol: d₃-(1-Hydroxycyclohexyl)-(4-methoxyphenyl)-acetonitrile (400.0 mg, 1.61 mmol) was reduced on an H-Cube™ continuous-flow hydrogenation reactor (Thales Nanotechnology, Budapest, Hungary) equipped with a water reservoir for the generation of hydrogen gas and Raney Ni catalyst cartridge (eluent: 2.0M ammonia *in* ethanol, flow rate: 1 mL/min, temperature: 80°C, pressure: 80 bar) to yield the desired product, d₃-1-[2-amino-1-(4-methoxyphenyl)-ethyl]-cyclohexanol, as a clear colorless oil. Yield: 280 mg (69%). ¹H-NMR (CDCl₃) δ ppm: 1.05-1.80 (m, 10H), 2.59 (br s, 2H), 2.68 (t, 1H, 6.9Hz), 3.21 (m, 2H), 6.83 (d, 2H, J = 9.0Hz), 7.17 (d, 2H, J = 9.0Hz).

### Step 4

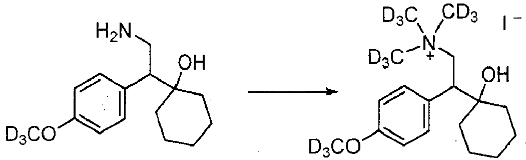

d₁₂-1-[2-Trimethylammonium-1-(4-methoxyphenyl)-ethyl]-cyclohexanol iodide: d₃-Iodomethane (0.4 mL, 6.34 mmol) and potassium carbonate (424mg, 3.0 mmol) were added at ambient temperature to a solution of *d*₃-1-[2-amino-1-(4-methoxyphenyl)-ethyl]-cyclohexanol (252 mg, 1.0 mmol) in anhydrous tetrahydrofuran (1.5 mL), and stirred at ambient temperature for 20 hours. The reaction mixture was diluted with tetrahydrofuran, filtered, and the filtrate was concentrated in vacuo to provide the product, d₁₂-1-[2-trimethylammonium-1-(4-methoxyphenyl)-ethyl]-cyclohexanol iodide, as a beige solid. ¹H-NMR (CD₃OD) δ: 0.90-1.80 (m, 10H), 3.19 (m, 1H), 4.00 (m, 2H), 6.93 (d, J = 8.1Hz, 2H), 7.43 (d, J = 8.1Hz, 2H).

### Step 5

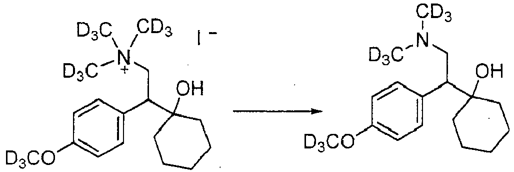

d₉-1-[2-Dimethylamino-1-(4-methoxyphenyl)-ethyl]-cyclohexanol-(d₉-venlafaxine): A solution of d₁₂-1-[2-trimethylammonium-1-(4-methoxyphenyl)-ethyl]-cyclohexanol iodide in 3-amino-1-propanol (1 mL) was heated at 170°C for 4 hours, cooled to ambient temperature, diluted with water and extracted with ethyl acetate. The combined organic layers were washed with brine, dried and concentrated under reduced pressure. The resulting residue was dissolved in 6N hydrochloric acid (5 mL), washed with ether. The aqueous layer was basified with 30% aqueous sodium hydroxide to pH = 11-12 and extracted with ethyl acetate. The organic extract was washed with brine, dried, and concentrated to afford *d*₉-venlafaxine (208 mg, 73%). ¹H-NMR (CDCl₃) δ ppm: 0.78-1.80 (m, 10H), 2.33 (dd, 1H, J = 12.0, 3.3 Hz), 2.96 (dd, 1H, J = 12.0, 3.3 Hz), 3.31 (t, 1H, J =12.0 Hz), 6.81 (d, 2H, J = 9.0Hz), 7.17 (d, 2H, J = 9.0Hz). MS (m/z): 287 (M+1)

### Step 6

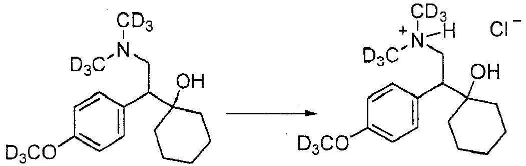

d₉-1-[2-Dimethylamino-1-(4-methoxyphenyl)-ethyl]-cyclohexanol hydrochloride - (d₉-venlafaxine hydrochloride): A solution of d₉-venlafaxine (63mg, 0.22 mmol) in ether (10 mL) was treated with 2N hydrochloric acid in ether (0.2 mL) at 0°C for 10 minutes. The white precipitate was collected by filtration, washed with ether, and dried in vacuo to provide d₉-venlafaxine hydrochloride salt (60mg, 85%). ¹H-NMR (CD₃OD) δ: 0.95-1.80 (m, 10H), 2.83(s, 6H), 3.04 (dd, 1H, J = 9.9, 5.4 Hz), 3.68 (m, 2H), 6.96 (d, 2H, J = 9.0 Hz), 7.30 (d, 1H, J = 9.0 Hz).

### EXPERIMENTAL METHODS

### Human Oral Pharmacokinetics

Human subjects received solutions of either 30 mg d₉-venlafaxine hydrochloride or 30 mg venlafaxine hydrochloride. Blood samples for determination of plasma concentration of venlafaxine, d₉-venlafaxine and their respective metabolites were collected pre-dose (within 60 minutes prior to dose administration) and at 15, 30, 45 minutes, 1, 1.5, 2, 2.5, 3, 3.5, 4, 8, 12, 24, 36, 48, 72 and 96 hours post-dose (18 samples in each treatment period). Total urine for measurement of parent drug and metabolites excreted in urine was collected pre-dose and for the intervals 24 hours post-dose in each treatment period. The urine concentration was pooled for analysis of the total amount of parent and metabolite excreted over the 24-hour period. Changes in various pharmacokinetic parameters of d₉-venlafaxine and its respective metabolites were observed as compared to the non-isotopically enriched venlafaxine and corresponding metabolites.

Changes in interpatient variability are reported below in Table 1. Relative standard deviation (RSD) in each relevant parameter in the deuterated group was divided by RSD in the nondeuterated group, subtracted from 1, and multiplied by 100 to yield percent decrease in interpatient variability. In Table 1, + indicates ≥ 15% reduction in interpatient variability, ++ indicates ≥ 20% reduction in interpatient variability, and +++ indicates ≥ 40% reduction in interpatient variability.

**Table 1.**

| **Measured Parameter** | **Reduction in Inter-Patient Variability** |
|---|---|
| Plasma Half Life (T_{1/2}) | ++ |
| Time at Maximum Plasma Concentration (Tₘₐₓ) | + |
| Maximum Plasma Concentration (Cₘₐₓ) | +++ |
| Time vs. Concentration Area Under the Curve (AUC) | ++ |

Reduction in incidence of side effects (adverse events) are reported below in Table 2, in which + indicates ≥ 25% reduction in the incidence of the given side effect, ++ indicates ≥ 50% reduction in the incidence of the given side effect, and +++ indicates ≥ 75% reduction in the incidence of the given side effect. NI indicates that there was a net increase in the incidence of a given side effect. The total incidence of side effects was over 35% lower in the group taking d₉-venlafaxine compared to the venlafaxine group.

**Table 2.**

| Adverse Event | Percent Reduction in Side-Effect Incidence, d₉-Venlafaxine HCl vs. Venlafaxine HCl |
|---|---|
| Drowsiness | NI |
| Dry Mouth | ++ |
| Fatigue | + |
| Headache | ++ |
| Lightheadedness | + |
| Nausea | NI |
| Emesis | +++ |

### In vitro Liver Microsomal Stability Assay

Liver microsomal stability assays were conducted at 1 mg per mL liver microsome protein with an NADPH-generating system in 2%NaHCO₃ (2.2 mM NADPH, 25.6 mM glucose 6-phosphate, 6 units per mL glucose 6-phosphate dehydrogenase and 3.3 mM MgCl₂). Test compounds were prepared as solutions in 20% acetonitrile-water and added to the assay mixture (final assay concentration 5 microgram per mL) and incubated at 37°C. Final concentration of acetonitrile in the assay were <1%. Aliquots (50µL) were taken out at times 0, 15, 30, 45, and 60 minutes, and diluted with ice cold acetonitrile (200 µL) to stop the reactions. Samples were centrifuged at 12000 RPM for 10 minutes to precipitate proteins. Supernatants were transferred to microcentrifuge tubes and stored for LC/MS/MS analysis of the degradation half-life of the test compounds. It has thus been found that the compounds disclosed herein which have been tested in this assay showed an increase of 10% or more in the degradation half-life, as compared to the non-isotopically enriched drug. For example, the degradation half-life of d₉-venlafaxine is increased about 200% as compared to non-isotopically enriched venlafaxine.

It is expected that other compounds which show an increase in degradation half-life in the microsomal assay will also show reduced interpatient variability. Examoles of such compounds are given in Table 3 below, in which D represents an increase in degradation half-life of 0 to 50% over the non-isotopically enriched compound, C represents an increase in degradation half-life of 51% to 100%, Brepresents an increase in degradation half-life of 101% to 150%, and A represents an increase in degradation half-life of 151 to 200% or more.

**Table 3.**

| **Drug** | **Deuterated Analogue** | **Increase** |
|---|---|---|
| Almotriptan | 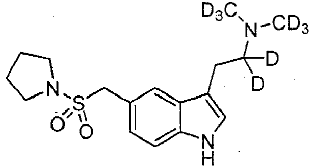 | D |
| Almotriptan | 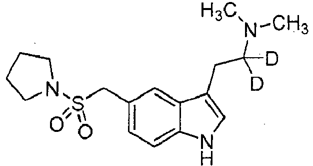 | D |
| Atomoxetine | 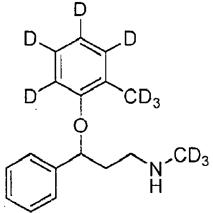 | B |
| Atomoxetine | 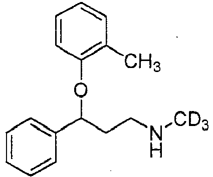 | D |
| Bosetan | 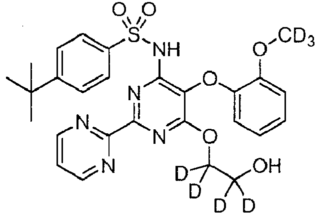 | D |
| Budesonide | 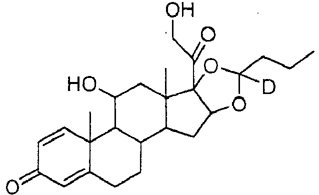 | A |
| Carisoprodol | 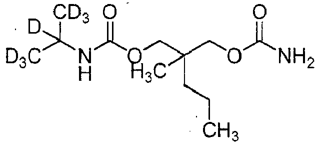 | D |
| Clarithromycin | 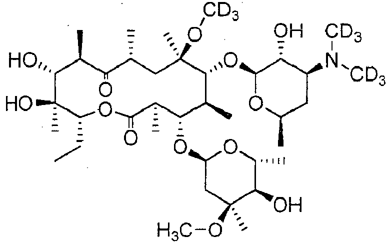 | B |
| Clarithromycin | 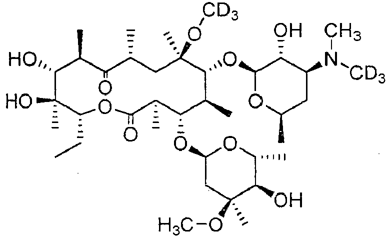 | C |
| Clarithromycin | 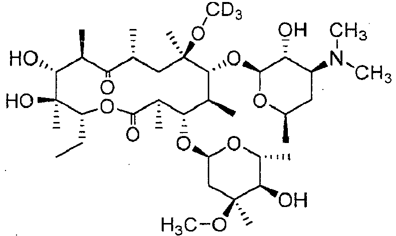 | C |
| Clemastine | 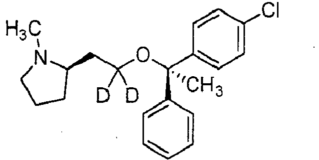 | D |
| Codeine | 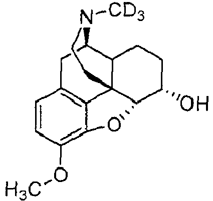 | D |
| Cyclophosphamide | 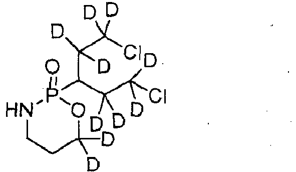 | D |
| Cyclophosphamide | 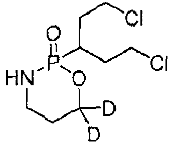 | D |
| Ethambutol | 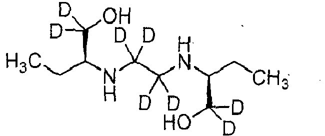 | D |
| Fentanyl | 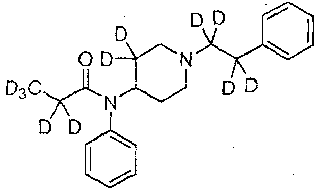 | D |
| Fentanyl | 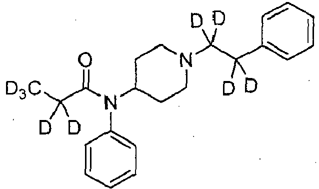 | D |
| Fingolimod | 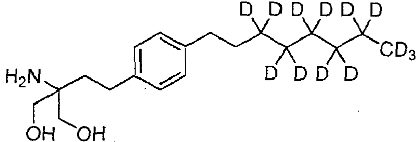 | D |
| Fluoxetine | 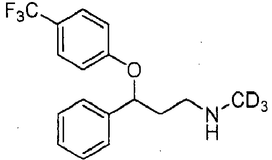 | D |
| Hydrocodone | 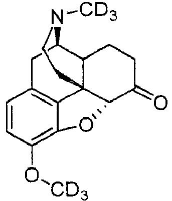 | D |
| Hydrocondone | 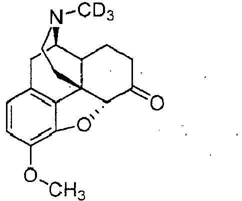 | D |
| Ibuprofen | 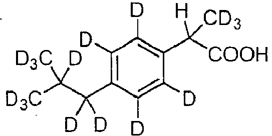 | B |
| Ilaprazole | 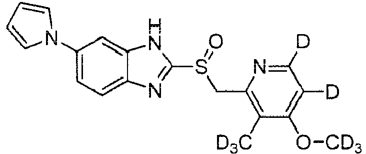 | D |
| Ketamine | 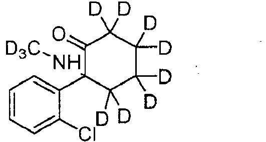 | B |
| Linezolid | 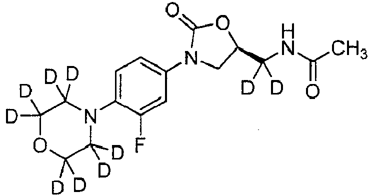 | D |
| Linezolid | 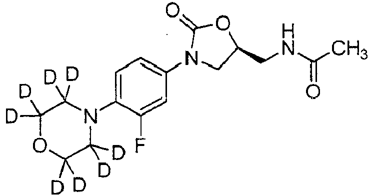 | D |
| Losartan | 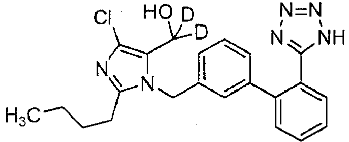 | B |
| Losartan | 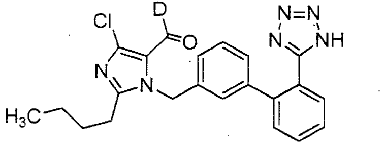 | C |
| Maraviroc | 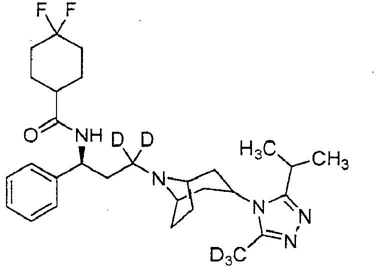 | D |
| Meperidine | 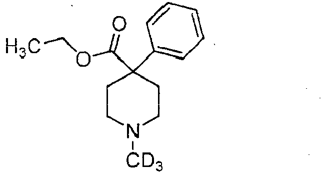 | D |
| Metaxalone | 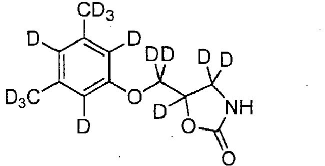 | D |
| Metoprolol | 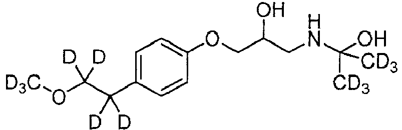 | D |
| Metoprolol | 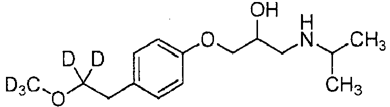 | D |
| Metoprolol | 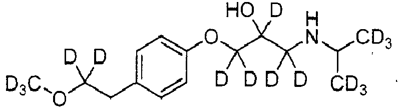 | D |
| Metoprolol | 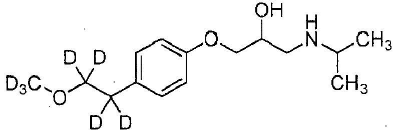 | D |
| Morphine | 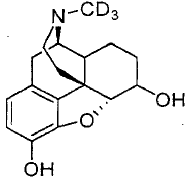 | D |
| Mosapride | 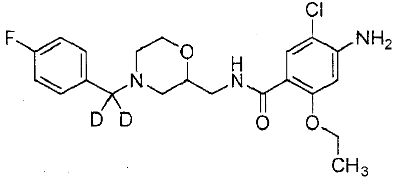 | D |
| Nicotinamide | 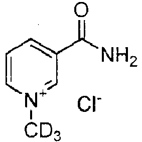 | D |
| Nicotinamide | 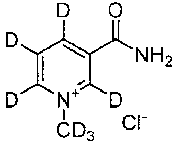 | D |
| Nicotinamide | 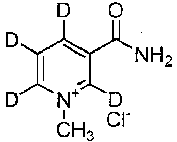 | D |
| Omeprazole | 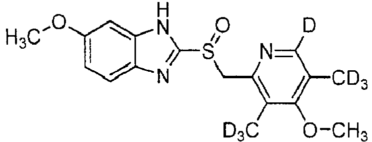 | D |
| Omeprazole | 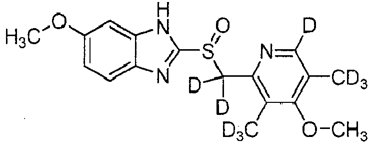 | D |
| Omeprazole | 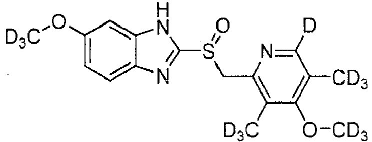 | D |
| Olanzapine | 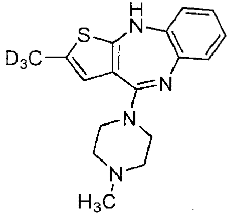 | D |
| Olanzapine | 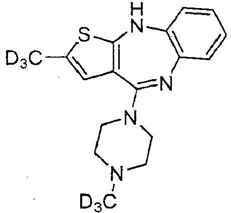 | D |
| Oxycodone | 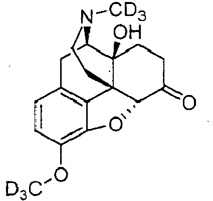 | D |
| Oxycodone | 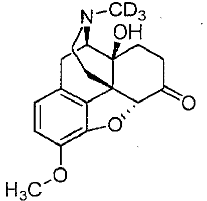 | D |
| Pantoprazole | 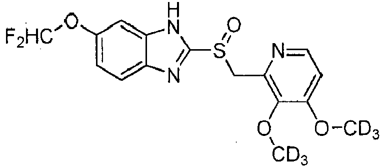 | A |
| Pantoprazole | 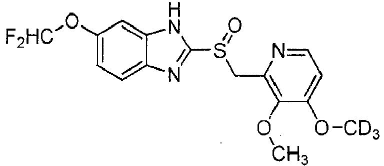 | B |
| Pantoprazole | 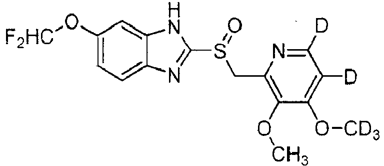 | B |
| Pantoprazole | 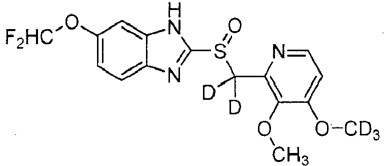 | C |
| Phenylephrine | 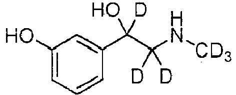 | B |
| Phenylephrine | 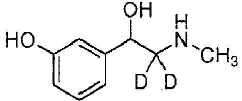 | B |
| Phenylephrine | 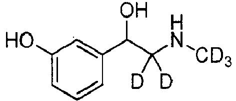 | B |
| Phenylephrine | 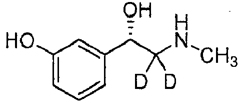 | B |
| Phenylephrine | 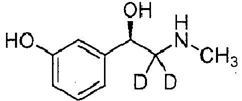 | B |
| Phenylephrine | 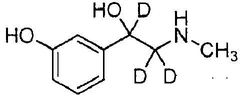 | B |
| Phenylephrine | 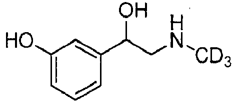 | D |
| Pirfenidone | 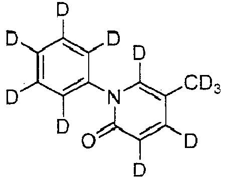 | D |
| Pirfenidone | 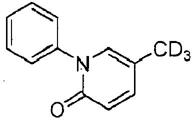 | D |
| Propanolol | 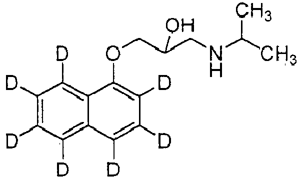 | D |
| Propanolol | 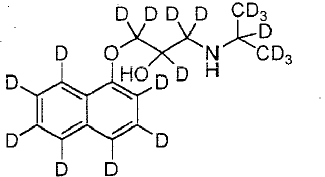 | D |
| Ranolazine | 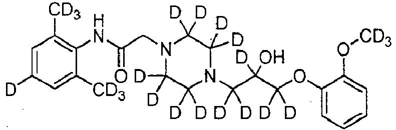 | D |
| Resatorvid | 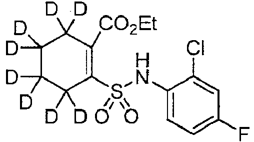 | D |
| Rimonabant | 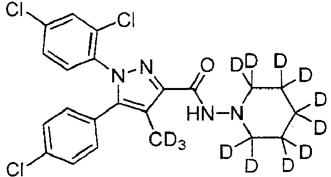 | D |
| Rimonabant | 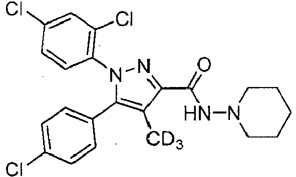 | D |
| Sitaxsentan | 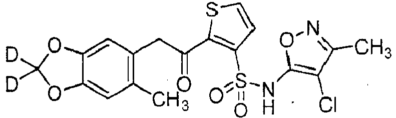 | D |
| Sumatriptan | 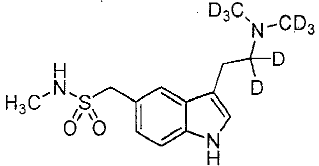 | A |
| Sumatriptan | 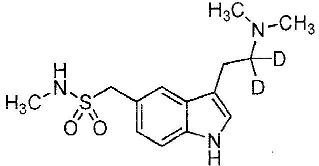 | B |
| Tenatoprazole | 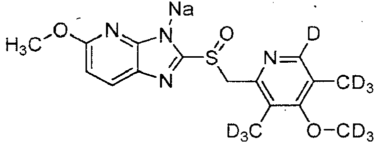 | C |
| Tenatoprazole | 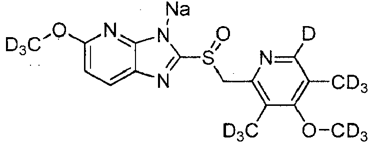 | D |
| Tramadol-Cis | 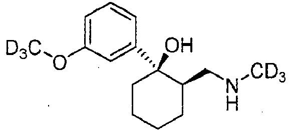 | B |
| Tramadol-Cis | 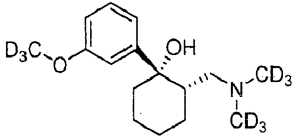 | B |
| Tramadol-Cis | 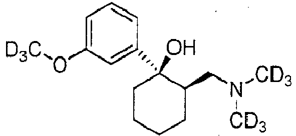 | B |
| Tramadol-Cis | 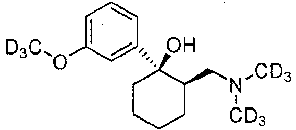 | C |
| Tramadol-Cis | 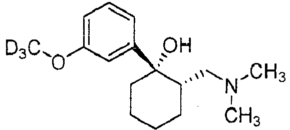 | C |
| Tramadol-Cis | 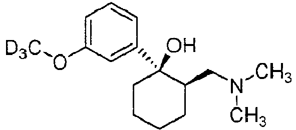 | D |
| Tramadol-Cis | 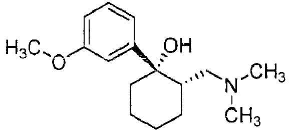 | D |
| Tramadol-trans | 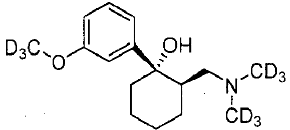 | D |
| Trazodone | 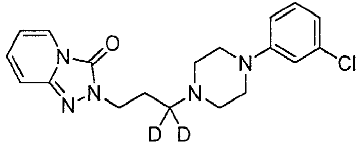 | D |
| Zafirlukast | 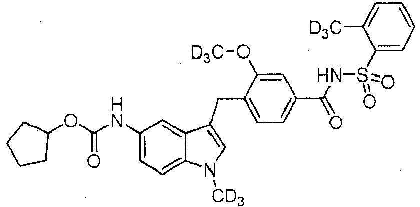 | D |
| Zolpidem | 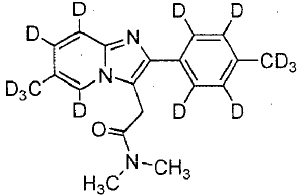 | C |
| Zolpidem | 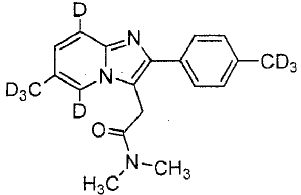 | C |
| Zolpidem | 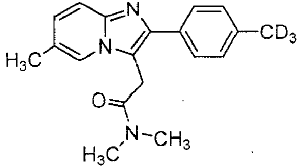 | D |
| Zolpidem | 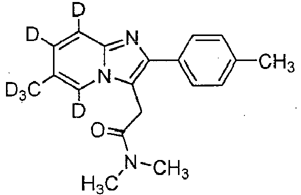 | D |

From the foregoing description, one skilled in the art can easily ascertain the essential characteristics of this invention, and without departing from the spirit and scope thereof, can make various changes and modifications of the invention to adapt it to various usages and conditions.
1. A method of reducing inter-patient variability in a drug treatment, comprising;
   i. identifying inter-patient variability in said drug treatment;
   ii. preparing a deuterated analog of said drug; and
   iii. using said deuterated analog in said drug treatment to reduce inter-patient variability of said drug treatment.
2. The method as recited in claim 1 wherein said deuterated analog has deuteration at one or more sites metabolized by a cytochrome P₄₅₀.
3. The method as recited in claim 2 wherein said cytochrome P₄₅₀ is a polymorphically expressed cytochrome P₄₅₀.
4. The method as recited in claim 3 wherein said polymorphically expressed cytochrome P₄₅₀ is selected from the group consisting of CYP3A4, CYP2D6 and CYP2C19.
5. The method as recited in claim 1 wherein said interpatient variability is in the frequency or severity of side effects experienced.
6. The method as recited in claim 1 wherein said interpatient variability is in the half-life (t_{½}) of a drug in the plasma.
7. The method as recited in claim 1 wherein said interpatient variability is in the maximum plasma concentration (Cₘₐₓ) of a drug in the plasma.
8. The method as recited in claim 1 wherein said interpatient variability is in the time at maximal plasma concentration (Tₘₐₓ) of a drug in the plasma.
9. The method as recited in claim 1 wherein said interpatient variability is in the area under the time-versus-concentration curve (AUC) of a drug in the plasma.
10. The method as recited in claim 1 wherein said interpatient variability is in the metabolite profile of the drug.
11. The method as recited in claim 1 wherein said drug is venlafaxine.
12. The method as recited in claim 11 wherein using said deuterated analog yields a reduced inter-patient variability in plasma half life (t_{½}) of 20% or more compared to non-deuterated venlafaxine.
13. The method as recited in claim 11 wherein using said deuterated analog yields a reduced inter-patient variability in maximum plasma concentration (Cₘₐₓ) of 30% or more compared to non-deuterated venlafaxine.
14. The method as recited in claim 11 wherein using said deuterated analog yields a reduced inter-patient variability in time at maximal plasma concentration (Tₘₐₓ) of 15% or more compared to non-deuterated venlafaxine.
15. The method as recited in claim 11 wherein using said deuterated analog yields a reduced inter-patient variability in plasma area under the time-versus-concentration curve (AUC) of 20% or more compared to non-deuterated venlafaxine.
16. The method as recited in claim 11 wherein using said deuterated analog yields a reduction in the incendence of one or more side effects.
17. The method as recited in claim 16 wherein using said deuterated analog yields at least 25% fewer side effects overall than non-deuterated venlafaxine.
18. The method as recited in claim 16 wherein the side effect is selected from the group consisting of drowsiness, dry mouth, fatigue or somnolence, headache, lightheadedness, nausea, emesis, mydriasis, anxiety, nervousness, and insomnia, dizziness, anxiety, asthenia, sweating, anorexia, weight gain, activation of mania/hypomania, and suicidal ideation.
19. A method of reducing interpatient variability in a population of patients taking a drug comprising
   i. identifying a population of patients having interpatient variability; and
   ii. administering to a population of patients a deuterated analog of a drug.
20. A deuterated analog of venlafaxine having a reduced inter-patient variability in plasma half life of 20% or more compared to non-deuterated venlafaxine.
21. A deuterated analog of venlafaxine having a reduced inter-patient variability in maximum plasma concentration in humans of 30% or more compared to non-deuterated venlafaxine.
22. A deuterated analog of venlafaxine having a reduced inter-patient variability in maximum plasma concentration in humans of 40% or more compared to non-deuterated venlafaxine.
23. A deuterated analog of venlafaxine having a reduced inter-patient variability time at maximal plasma concentration in humans of 15% or more compared to non-deuterated venlafaxine.
24. A deuterated analog of venlafaxine having a reduced inter-patient variability in plasma area under the time-versus-concentration curve 20% or more compared to non-deuterated venlafaxine.
25. A deuterated analog of venlafaxine which causes at least 25% fewer side effects than non-deuterated venlafaxine.

## Claims

1. A deuterated analog of venlafaxine for use in the treatment of a disease or condition selected from the group consisting of anxiety disorder, generalized anxiety disorder, depression, post-traumatic stress disorder, obsessive-compulsive disorder, panic disorder, hot flashes, senile dementia, migraine, hepatopulmonary syndrome, chronic pain, nociceptive pain, neuropathic pain, painful diabetic retinopathy, bipolar depression, obstructive sleep apnea, psychiatric disorders, premenstrual dysphoric disorder, social phobia, social anxiety disorder, urinary incontinence, anorexia, bulimia nervosa, obesity, ischemia, head injury, calcium overload in brain cells, drug dependence, and premature ejaculation.

2. The deuterated analog of venlafaxine for use as recited in claim 1 wherein said deuterated analog has deuteration at one or more sites metabolized by a cytochrome P₄₅₀.

3. The deuterated analog of venlafaxine for use as recited in claim 2 wherein said cytochrome P₄₅₀ is a polymorphically expressed cytochrome P₄₅₀ selected from the group consisting of CYP3A4, CYP2D6 and CYP2C19.

4. The deuterated analog of venlafaxine for use as recited in claim 1 wherein using said deuterated analog yields a reduced inter-patient variability in plasma half life (t_{½}) of 20% or more compared to non-deuterated venlafaxine.

5. The deuterated analog of venlafaxine for use as recited in claim 1 wherein using said deuterated analog yields a reduced inter-patient variability in maximum plasma concentration (Cₘₐₓ) of 30% or more compared to non-deuterated venlafaxine.

6. The deuterated analog of venlafaxine for use as recited in claim 1 wherein using said deuterated analog yields a reduced inter-patient variability in time at maximal plasma concentration (Tₘₐₓ) of 15% or more compared to non-deuterated venlafaxine.

7. The deuterated analog of venlafaxine for use as recited in claim 1 wherein using said deuterated analog yields a reduced inter-patient variability in plasma area under the time-versus-concentration curve (AUC) of 20% or more compared to non-deuterated venlafaxine.

8. The deuterated analog of venlafaxine for use as recited in claim 1 wherein using said deuterated analog yields a reduction in the incidence of one or more side effects.

9. The deuterated analog of venlafaxine for use as recited in claim 8 wherein using said deuterated analog yields at least 25% fewer side effects overall than non-deuterated venlafaxine.

10. The deuterated analog of venlafaxine for use as recited in claim 8 wherein the side effect is selected from the group consisting of drowsiness, dry mouth, fatigue or somnolence, headache, lightheadedness, nausea, emesis, mydriasis, anxiety, nervousness, and insomnia, dizziness, anxiety, asthenia, sweating, anorexia, weight gain, activation of mania/hypomania, and suicidal ideation.
